# EUROPEAN PATENT APPLICATION

(11) **EP 4 169 486 A1**
(43) Date of publication of application: **26.04.2023**
(21) Application number: 22203516.4
(22) Date of filing: 25.10.2022
(51) Int. Cl.: A61F 2/30, A61F 2/34, B22F 10/28

(54) **POROUS STRUCTURE PLACEMENT CONFIGURED FOR MANUFACTURING**

(30) Priority: 25.10.2021 US 202163271436 P
(71) Applicant: Howmedica Osteonics Corporation, Mahwah, New Jersey 07430 (US)
(72) Inventor: JACONELLI, Anthony, P31YN15 Ballincollig, co. Cork (IE); MCAULIFFE, Shane, Mallow, co. Cork (IE); KENNEALLY, Paddy, P61 XT72 Kilworth, co. Cork (IE)
(74) Representative: Regimbeau

(57) **Abstract**

A computer-generated component file for fabricating an orthopedic implant is prepared. First and second select sections of an initial implant model (108) of a computer-aided design model are set to first and second model porous sections (130). A remaining section (110) of the initial implant model is left. All regions defining the first and the second select sections are spaced not more than a preset distance from a patient-specific bone model (100) of the computer-aided design model as measured uniformly. The first and the second model porous sections are merged with a remaining section of the initial implant model to form at least a portion of a final implant model (150). The final implant model is stored in a component file configured to be accessed by a computer-aided manufacturing machine for use in fabricating the orthopedic implant. At least a portion of the orthopedic implant corresponds to the final implant model.

## Description

### CROSS-REFERENCE TO RELATED APPLICATION

The present application claims the benefit of the filing date of U.S. Provisional Application No. 63/271,436 filed October 25, 2021, the entirety of the disclosure of which is hereby incorporated herein by reference.

### FIELD

The present invention generally relates to orthopedic implant fabrication optimization, and in particular relates to a system and process for improving the efficiency of the manufacturing of porous orthopedic implants while maintaining osseointegration capabilities of such implants.

### BACKGROUND

Some varieties of orthopedic implants act as prostheses replacing a removed portion of bone. For example, where a portion of a bone is injured or diseased, such portion may be removed and replaced by an implant of similar size and shape. Such implants may benefit from having hard, smooth surfaces, particularly where the removed portion of the bone forms part of a joint. However, smooth surfaces tend not to be conducive to fixation of the implant into the bone. Implants may therefore be provided with a rough or porous side for contacting the cut surface of the bone to facilitate growth of the bone into the implant.

Certain manufacturing difficulties are associated with implant designs having a portion that is porous or otherwise different in microstructure or material from another portion of the implant. For example, an implant design having planned porous regions near through holes or other exposed features may experience ablation due to poor heat dissipation during fabrication or lack structural integrity during or following fabrication the implant is produced using techniques such as additive manufacturing, e.g., powder bed fusion ("PBF") processes. Custom patient-matched implants can further complicate the design and manufacturing processes. Design choices intended to improve the ease of manufacture or strength of a component may conflict with design choices intended to improve osseointegration or fidelity to a given patient's anatomy, as these factors are usually considered separately. Improvements in implant designs and manufacturing strategies that reduce manufacturing difficulties or time with minimal compromise to goals such as osseointegration or anatomical fidelity are therefore needed.

### BRIEF SUMMARY

According to some aspects, an implant optimization process for providing these improvements and achieving these goals may begin with a digital implant model, which may be a computer-aided design (CAD) model, corresponding to the net shape of an orthopedic implant positioned relative to a digital model of a bone. For the purposes of placing osseointegrative structures or materials, a bone interface side of the implant model may be identified, e.g., automatically by CAD modeling software or manually by a user of such software via the software, as including all digital surfaces of the implant model that are exposed in a given direction that is at least generally toward the bone model, with some optional exclusions. A model of an interface layer of osseointegrative material or structures may be identified within the digital implant model as extending from the surfaces of the interface side along directions of interface between the digital implant and the digital model of the bone at various locations. The interface material layer, or portions of the interface side selected for conversion to osseointegrative structures or materials, may be excluded from certain areas. The exclusions may include areas more than a certain distance from the bone model, areas within a certain distance of separate model components, or areas within a certain distance of an opposing side of the implant model opposed to the interface side. The interface layer may be a porous layer. Porous material may be extended beyond the initial net shape of the implant model from the interface side, and transitional amounts of porosity, e.g., a gradient porosity, may be introduced to areas of the implant model near the interface layer.

In further aspects, the opposing side of the implant model, which in some arrangements may act as a support side, may be identified within the digital implant model as including all digital surfaces of the implant model exposed and facing toward a direction from which material will be built up in an additive manufacturing process for which the implant model is intended. An augmented layer, which in some arrangements may act as a support layer, may be added to the opposing side. Material corresponding to the support layer may be machined off of a component fabricated according to the implant model or a separate CAD model or computer-aided manufacturing (CAM) model that corresponds to such machined features.

In another aspect, a computer-generated component file for fabricating an orthopedic implant is prepared by a process. In the process, via the computer processor, first and second select sections of an initial implant model of a computer-aided design model may be set to first and second model porous sections and thereby leave a remaining section of the initial implant model, which may be in a form matching the form of the remaining section prior to the setting of the first and the second select sections. All regions defining the first and the second select sections may be spaced not more than a preset distance from a patient-specific bone model of the computer-aided design model, e.g., as measured or otherwise determined uniformly. In the process, the first and the second model porous sections may be merged, via the computer processor, with the remaining section of the initial implant model to form at least a portion of a final implant model of the computer-aided design model. The final implant model may be stored, via the computer processor, in a component file configured to be accessed by a computer-aided manufacturing machine for use in fabricating the orthopedic implant. In this manner, at least a portion of the orthopedic implant may correspond to the final implant model.

In some arrangements, the distances from regions defining each of the first and the second select sections to the patient-specific bone model to be compared to the preset distance may be measured or otherwise determined uniformly in various ways. In some arrangements, such distances may be distances along respective axes extending perpendicularly from each of the regions defining each of the first and the second select sections to respective closest points on the bone model that lie along the respective axes. In some other arrangements, such distances may be distances along respective axes extending perpendicularly from points on the bone model to respective closest points each of the regions defining each of the first and the second select sections that lie along the respective axes. In still other arrangements, such distances may be the shortest distances from each of the regions defining each of the first and the second select sections to respective closest points on the bone model. Still other ways, known to those skilled in the art and as described further herein, in which to determine the distances from regions defining each of the first and the second select sections to the patient-specific bone model to be compared to the preset distance are contemplated within the scope of the present disclosure.

In some arrangements according to any of the foregoing, , before the setting step and via the computer processor, the first and the second select sections may be removed from the initial implant model. In some such arrangements, the remaining section of the initial implant model may remain following the removal of the first and the second select sections.

In some arrangements according to any of the foregoing, the first and the second model porous sections may have a first model porous thickness. In some such arrangements, via the computer processor, a third select section of the initial implant model may be set to a third model porous section. With the third model porous section set, all regions defining the third select section may be spaced not more than the preset distance from the patient-specific bone model, e.g., as measured or otherwise determined uniformly. In some arrangements, the distances of such regions from the bone model may be determined in the similar manner as the distances from regions defining each of the first and the second select sections to the bone model, as described previously herein, and then compared to the preset distance. With the third model porous section set, first and second model implant thicknesses of the initial implant model taken along respective first and second lines through the first and the second select sections may include at least a preset minimum model continuous solid thickness and a third model implant thickness of the initial implant model taken along a third line through the third select section may include less than the preset minimum model continuous solid thickness. With the third model porous section set, the third model porous section may have a second model porous thickness equal to a difference between the third model implant thickness and the preset minimum model continuous solid thickness when the third model implant thickness is either one or both greater than zero and greater than the preset minimum model continuous thickness. In some such arrangements, the third model porous section having a second model porous thickness equal to a difference between the third model implant thickness and the preset minimum model continuous solid thickness when the third model implant thickness is greater than the preset minimum model continuous solid thickness or is otherwise zero. With the third model porous section set, the third model porous section may be merged along with the first and the second model porous sections, via the computer processor, with the remaining section of the initial implant model to form at least the portion of the final implant model.

In some arrangements according to any of the foregoing, in setting of any one or any combination of the first, the second, and the third model porous sections, instructions may be sent, via the computer processor, to a computer-aided design model to associate any one or any combination of a porosity, a pore size, and a pore density with the respective one or ones of the first, the second, and the third model porous sections of the initial implant model.

In some arrangements according to any of the foregoing,, before the third select section setting step and via the computer processor, the third select section may be removed from the initial implant model.

In some arrangements according to any of the foregoing, via the computer processor, an input instruction may be received. In some such arrangements, each of the removing, setting, and merging steps may occur automatically in response to the input instruction.

In some arrangements according to any of the foregoing, the first and the second select sections may include first and second digital surfaces contacting the patient-specific bone model.

In some arrangements according to any of the foregoing, before the setting step and via the computer processor, any regions of the initial implant model that are not within a porous thickness distance of any point on any surface of the initial implant model that is within an interface proximity of a bone model may be removed from the select sections.

In some arrangements according to any of the foregoing, via the computer processor, the model porous sections may be extended beyond the initial implant model.

In some arrangements according to any of the foregoing, via the computer processor, a boundary region of the initial implant model may be set. In some such arrangements, the boundary region may be contiguous with one or more, e.g., all, of the model porous sections as a region of gradient porosity.

In some arrangements according to any of the foregoing, via the computer processor, a model added thickness layer may have a predetermined model thickness to a side of the initial implant model facing away from the bone model.

In some arrangements according to any of the foregoing, a component may be fabricated, e.g., via additive or subtractive manufacturing processes known to those skilled in the art, according to the final implant model. In some such arrangements, at least some material from the component corresponding to the added thickness layer may be removed to define at least a portion of the orthopedic implant.

In another aspect, a portion of a bone may be replaced. In this process one or more bone portions may be removed from one or more bones to leave, respectively, a remaining first bone portion or a remaining first plurality of bone portions. In the process, an implant having a plurality of irregularly shaped porous portions and a plurality of irregularly shaped solid portions each separating at least portions of respective pairs of the plurality of irregularly shaped porous portions may be placed against only the respective remaining first bone portion or first plurality of bone portions such that none of the plurality of irregularly shaped porous portions includes a region located more than a preset distance from at least one bone portion of the respective remaining first bone portion or first plurality of bone portions. In such arrangements, the porous portions and the solid portions may define all bone-facing surface portions of the implant.

In another aspect, a computer-generated component file for fabricating an orthopedic implant may be prepared by a process. In this process, via a computer processor, a bone model, e.g., a patient-specific bone model, of a first bone portion or a first plurality of bone portions may be formed. In the process, via the computer processor, a first implant model may be formed with only porous regions and solid regions. In the process, all of the porous regions may be formed only at locations of the first implant model within a preset distance from the bone model. In the process, via the computer processor, the first implant model may be stored in a component file configured to be accessed by a computer-aided manufacturing machine, e.g., an additive (layer) manufacturing machine or a subtractive manufacturing machine such as but not limited to a computer numerically controlled (CNC) mill, for use in fabricating the orthopedic implant. In such arrangements, at least a portion of the orthopedic implant may correspond to the first implant model.

In some arrangements according to any of the foregoing, a model added thickness layer of a predetermined thickness may be extended to a side of the initial implant model facing away from the bone model.

In some arrangements according to any of the foregoing, a component may be fabricated according to the first implant model. In some such arrangements, a first portion of the component corresponding to at least part of the model added thickness layer may be machined away to expose a second portion of the component corresponding to at least one of the solid regions. In such arrangements, the one or more of the solid regions may correspond to the second portion of the component may be on the side of the initial implant model facing away from the bone model.

In some arrangements according to any of the foregoing, when forming the initial implant model, an axis extending through a surface facing the bone model and through the bone model may be defined. In some such arrangements, at least one and preferably all of the porous regions may be excluded from any region a predefined distance away from the axis.

In some arrangements according to any of the foregoing, when forming the initial implant model, the porous regions may be excluded from any region at least a predefined distance away from the side of the initial implant model facing away from the bone model.

In another aspect, a computing system may comprise at least one processor and at least one non-transitory, computer readable medium carrying instructions that, when read by the processor, would cause the processor to automatically perform the method of any of the foregoing aspects and arrangements.

In another aspect, an orthopedic implant may include a first implant structure corresponding to a first implant model of a computer-aided design model. The computer-aided design model may be prepared by a process. In this process, via a computer processor, a patient-specific bone model of a first bone portion or a first plurality of bone portions may be formed within the computer-aided design model. In the process, via the computer processor, the first implant model may be formed with porous regions and solid regions. In some arrangements, the porous regions may be formed only at locations of the first implant model within a preset distance from the bone model. In some arrangements, the first implant model may be formed with only the porous regions and the solid regions in which all of the porous regions may be formed only at locations of the first implant model within the preset distance from the bone model.

In some arrangements according to any of the foregoing, the first implant structure may be a custom implant fabricated at least in part by additive manufacturing using a component file comprising data corresponding to the first implant model.

In some arrangements according to any of the foregoing, the porous regions of the first implant model may be formed at all locations of the first implant model within the preset distance from the bone model with the exceptions of i) regions surrounding digital holes corresponding to holes configured for receiving shafts of separate objects separate from the first implant structure and ii) regions within a minimum continuous solid thickness zone of the first implant model. In some such arrangements, the minimum continuous solid thickness zone may have a boundary on a digital opposing surface of the first implant model opposite a digital bone-facing surface of the first implant model facing the bone model. In some such arrangements, the minimum continuous solid thickness zone may have a thickness corresponding to a minimum continuous solid thickness preset within the first implant model.

### BRIEF DESCRIPTION OF THE DRAWINGS

FIG. 1 is a perspective view of an initial digital implant model positioned on a digital bone model.
FIG. 2 is a perspective view of a digital implant model derived from the initial digital implant model of FIG. 1 with separate model components extending therethrough.
FIG. 3 is a perspective view of the implant model of FIG. 2 with holes defined therethrough for receiving the separate model components shown in FIG. 2.
FIG. 4 is a perspective view of the implant model of FIG. 3 with a digital reference post extending therethrough in a vertical direction.
FIG. 5 is a perspective view of the implant model of FIG. 3 with a projection extending from a bone-facing-side thereof.
FIG. 6 is a perspective view of a digital bone-facing surface prepared from the implant model of FIG. 3.
FIG. 7 is a perspective view of the bone model with axes extending along directions of contact with the digital bone-facing surface of FIG. 6 at several locations.
FIG. 8 is a perspective view of the digital bone-facing surface of FIG. 6 relative to the separate model components of FIG. 2.
FIG. 9 is a perspective view of a digital modified implant model in which an interface model derived from the digital bone-facing surface of FIG. 8 has been applied to the implant model of FIG. 3.
FIG. 10 is a cross-sectional view of boundary regions around a portion of the interface model of FIG. 9.
FIG. 11 is a perspective view of a further modified implant model that includes the modified implant model of FIG. 9, boundary regions added to the interface model, and a projection extending from a support side thereof.
FIG. 12 is a perspective view of an added thickness layer defined along the projection from the further modified implant model of FIG. 11.
FIG. 13 is a cutaway perspective view of the added thickness layer of FIG. 12 applied to and positioned relative to the further modified implant model of FIG. 11.
FIG. 14 is a perspective view of the added thickness layer of FIG. 12 applied to the further modified implant model of FIG. 11.
FIG. 15A is a flowchart of an osseointegration optimization process.
FIG. 15B is a flowchart of a structural optimization process.
FIG. 16 is a flowchart of a digital thread process for orthopedic implant design, optimization, and production.

### DETAILED DESCRIPTION

The present disclosure includes reference to processes carried out on a computer, with the illustrated example presenting steps carried out within a building application such as computer modeling or computer aided design ("CAD") software, e.g., Ntopology, NX, Solidworks, or any substantial equivalent in which a user may interface with the building application as described, e.g., in U.S. Patent No. 10,596,660, the disclosure of which is hereby incorporated herein by reference. However, any or all of the steps presented may optionally be carried out without any graphical presentation, particularly where such steps are carried out automatically by a computer. Any step or process in this disclosure or any combination thereof may, unless specified otherwise, be carried out by a human user of a computer carrying suitable CAD software, by a computing device having a processor and a non-transitory, computer readable medium carrying instructions that, when read by the processor, cause the processor to execute such steps or processes automatically, or by a human user in cooperation with such a computing device.

The computer-based design processes described herein can be used to create one or more manufacturing models that may be fabricated as physical products. Any fabrication method, including additive manufacturing, subtractive manufacturing, or a combination thereof, may be used as suitable for the size and shape of the output, meaning the final desired fabricated component, and the intended material or materials. An example includes using an additive manufacturing process as at least a first part of the fabrication process. In some arrangements, the additive manufacturing process may be, e.g., electron beam melting ("EBM"), selective laser sintering ("SLS"), selective laser melting ("SLM"), binder jetting, or blown powder fusion for use with metal powders.

When additive manufacturing by a PBF process such as EBM, SLM, or SLS, a first layer of metal powder is deposited onto a substrate and then scanned with a high energy beam so as to sinter or melt the powder and create a portion of one or more predetermined physical porous geometries. Successive layers of the metal powder are then deposited onto previous layers of the metal powder and also respectively scanned with the high energy beam prior to the deposition of subsequent layers of the metal powder. The scanning and depositing of successive layers of the metal powder continues the building process of the predetermined physical porous geometries. Such continuation of the building process refers not only to a continuation of a predetermined physical porous geometry from a previous layer but also a beginning of a new predetermined physical porous geometry as well as or instead of the completion of a predetermined physical porous geometry, depending on the desired characteristics of the structure or structures to be fabricated.

The structures formed using this process may be partially porous and, if desired, have interconnecting pores to provide an interconnecting porosity. In some arrangements, the physical porous geometries may be defined by physical struts connected at vertices corresponding to digitized nodes within a CAD or other modeling program. The powder and thus the additively printed porous portion or portions preferably may be made of any one or any combination of cobalt chrome alloy, titanium or alloy, stainless steel, niobium, tantalum, nitinol, zirconium, and magnesium. Additionally or in the alternative, polymers, including either or both or bioresorbable polymers and non-bioresorbable polymers may be used as additive manufacturing material. Thus, a mixture of desired mixed materials may be employed.

The high energy beam preferably may be an electron beam (e-beam) or laser beam and may be applied continuously to the powder or pulsed at a predetermined frequency. In some arrangements, the use of a laser or e-beam melting process may preclude the requirement for subsequent heat treatment of the structure fabricated by the additive manufacturing process, thereby preserving the initial mechanical properties of the additively manufactured porous portion. The high energy beam is emitted from a beam-generating apparatus to heat the metal powder sufficiently to sinter or at least partially melt the metal powder. High energy beam generation equipment for manufacturing such structures may be one of many currently available including the "Concept laser M2 Cusing" machines, GE Concept M2 Cusing Gen 2 machines, GE Arcam Q10 machines, 200W M2 Cusing (series 3), kW M2 Cusing (Series 3), Dual kW M2 Cusing (Series 5) MCP REALIZER, the EOS M270, TRUMPF TRUMAFORM 250, the ARCAM EBM S12 and Q10 machine, and the like. These machines are listed by way of example only. Thus, this list suggests what machines may have suitable capabilities for the processes described herein, but any other machines having similar capabilities may be used instead. The beam generation equipment may also be a customproduced laboratory device.

The pore density, pore size and pore size distribution may be controlled from one location to another. It is important to note that successive powder layers may differ in porosity by varying factors used for high energy beam scanning of powder layers. Additionally, the porosity of successive layers of powder may be varied by either creating a specific type of unit cell or manipulating various dimensions of a given unit cell. In some arrangements, the porosity may be a gradient porosity throughout at least a portion of the fabricated structure. The beam generation equipment may be programmed to proceed in a random generated manner to produce an irregular porous construct but with a defined level of porosity. Pseudo-random geometries may be formed by applying a perturbation to the vertices of porous geometries when preparing model build structures corresponding to the 3D structure to be fabricated. In this manner, the shapes and sizes of the physical porous geometries may be randomized.

In some arrangements, additively manufactured porous structures may be in the form of overlapping lines of solidified powder as disclosed in U.S. Patent No. 7,537,664, the disclosure of which is hereby incorporated by reference herein. In some arrangements, additively manufactured porous structures may be in the form of cellular structures defined by repeating formed porous geometries corresponding to digitized unit cells as disclosed in U.S. Patent Nos. 10,525,688 and 9,180,010, the disclosures of which are hereby incorporated by reference herein. In some arrangements, additively manufactured porous structures may be in the form of a mesh or chainmail as disclosed in U.S. Patent No. 10,596,660 and U.S. Patent No. 10,888,362, the disclosure of which is hereby incorporated by reference herein as if fully set forth herein. In some arrangements, additively manufactured structures may be formed to have flanges in the manner as disclosed in U.S. Patent No. 10,456,262, the disclosure of which is hereby incorporated by reference herein.

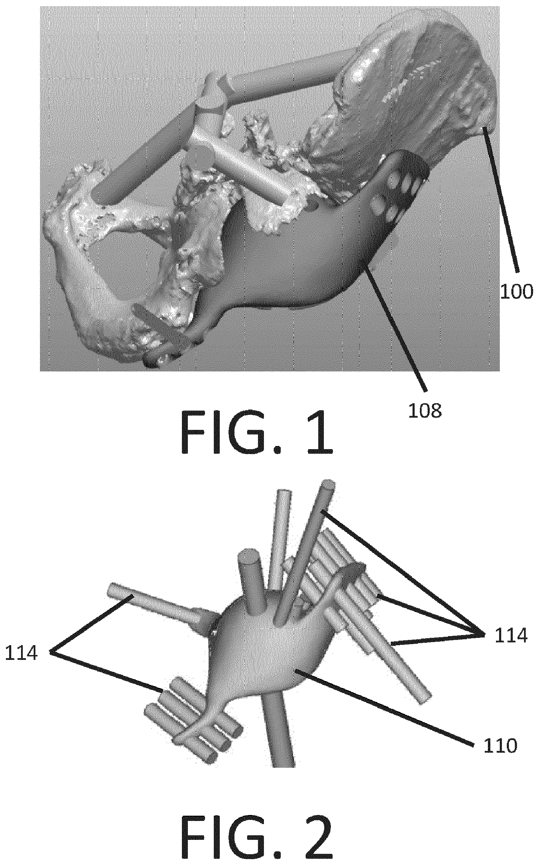

Referring now to FIG. 1, an implant design process may begin with input data. In the illustrated example, input data includes a scanned bone model 100 of a pelvis and a net shape implant model 108 for an acetabular implant. However, the processes and devices described herein may be applied to any type of bone and corresponding orthopedic implant. Bone model 100 is presented here by way of example, but input data may alternatively or additionally include an orthopedic model including representations of, in addition to the bone, soft tissue such as any one or any combination of cartilage, joint capsules, connective tissue such as either one or both of tendons and ligaments, muscle, and other soft tissue. In further alternatives, the input model may only include representations of soft tissues such as any of the foregoing soft tissues or combinations thereof. Bone model 100 will be discussed throughout this disclosure by way of example, but all uses for and operations upon bone model 100 may be performed additionally or instead with soft tissue models or orthopedic models that include both bone and soft tissue.

Scanned bone model 100 can be acquired in any way known for acquiring digital images of a bone within a patient's body, such as, for example, computerized tomography ("CT") or magnetic resonance imaging ("MRI"). Bone model 100 may be initially acquired in a preoperative or post-operative state. If acquired preoperatively, bone model 100 may be manipulated as necessary to resemble a planned postoperative state in which the implant being designed is implanted upon the bone.

Initial implant model 108 is a digital model having a shape and dimensions of a physical implant being designed but, unless desired, does not include any representation of material or structural variations, such as differences in porosity, throughout the implant. Initial implant model 108 may be off-the-shelf or patient-specific. If patient-specific, initial implant model 108 may be constructed entirely or at least partially by reference to the patient's bone or bones, such as by conforming the initial implant model to a difference between the post-resection state of bone model 100 and either an ideal shape or a preoperative bone model. For example, if the bone from which bone model 100 was derived already had significant preoperative deformities, initial bone model 108 may be shaped according to a difference between the planned post-resection state of bone model 100 and a theoretical ideal shape.

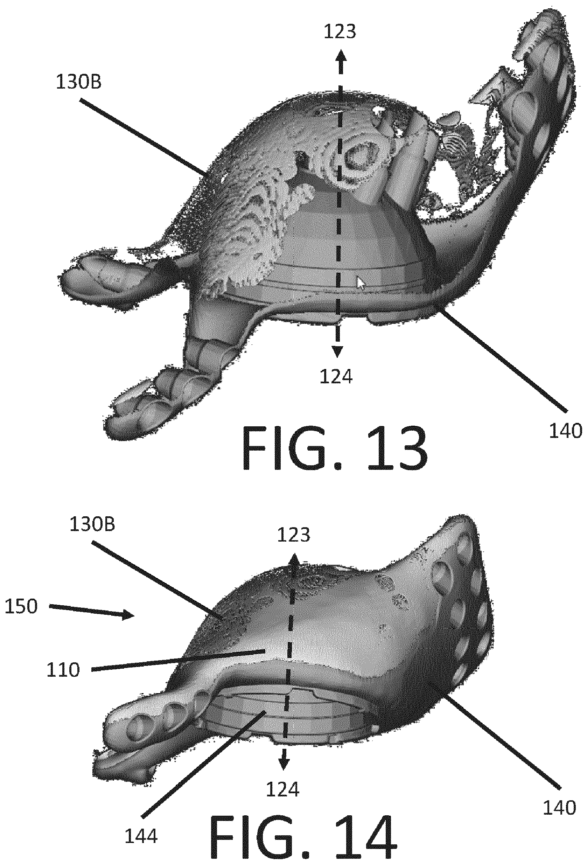

With reference to FIGS. 1 and 2, an optimization process for the implant includes making modifications to body portion 110 that corresponds to a geometry that will be constructed from a default or bulk material when optimized model 150 (see FIG. 14) is manufactured. Body portion 110 may begin as or be derived from initial implant model 108. That is, initial implant model 108 may be received by a worker or computer at a beginning of an optimization process, and body portion 110 manipulated throughout the operations described below either may be the initial implant model 108 itself or a model of generally similar geometry, dimensions, and placement as the initial implant model 108.

Body portion 110 typically represents a portion of the output that will have the implant's base material type such as, for example, titanium, cobalt chrome, stainless steel, or PEEK and structure, though special features or local variations may be manually added to the body portion at any time if desired, such as before, after, or between any of the steps described herein. Such base material may be any biocompatible material of sufficient strength and durability for the type of implant being designed, such as, variously, metals or metal alloys, with titanium, stainless steel, or Nitinol being some examples, ceramics, or polymers, with high-density polyethylene ("HDP"), polymethylmethacrylate ("PMMA"), or polyetheretherketone ("PEEK") being some examples. Features of the implant being designed other than such features as represented by body portion 110 may be constructed of the same material as or of different material than the material of the body portion features, and may be of the same porosity or of differing porosity.

As shown in FIG. 2, also input are separate model components 114, which include the shape, size, and location, or more commonly the diameter and trajectory, of bores that must extend through the implant being designed. Separate model components 114 do not necessarily correspond to features of the patient's anatomy or contours intended to be created by the implant being designed, though the separate model components may be placed with the patient's anatomy and the resulting contour in mind. Instead, separate model components 114 generally indicate the diameters and trajectories of screws, nails, or other fasteners, or rods, wires, or any other hardware that may be received through the implant being designed, or holes intended to receive such hardware.

Initial implant model 108 may be provided in a state already including holes corresponding to separate model components 114. Alternatively, initial implant model 108 may be provided without holes corresponding to separate model components 114, and such holes may be created within body portion 110.

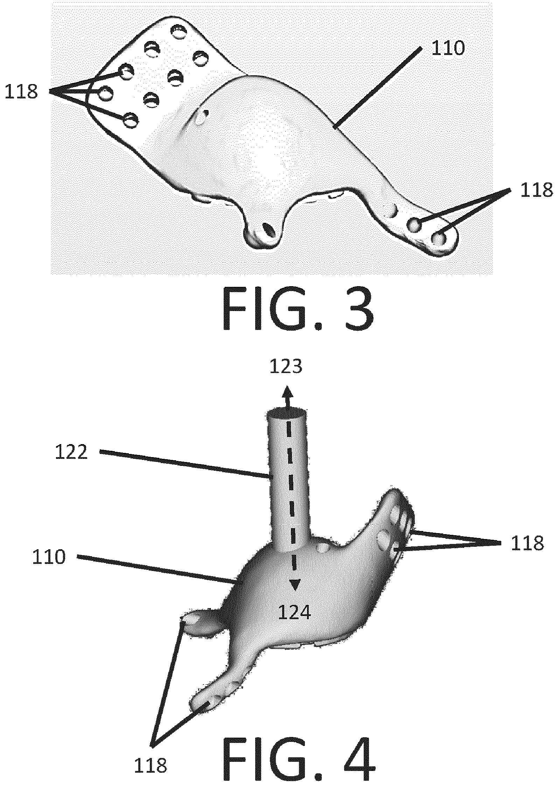

At a modeling stage as illustrated in FIG. 3, body portion 110 includes holes 118 corresponding in size, shape, and location to separate model components 114 where the separate model components pass through the body portion. If initial implant model 108 is input without holes for separate model components 114, holes 118 may be generated in body portion 110 through a Boolean operation where the separate model components 114 intersect the geometry of the initial implant model or the body portion.

As shown in FIG. 4, a vertical reference 122, which may be in the form of a digital post, may be defined relative to body portion 110, or vice versa. Vertical reference 122 extends along an axis relative to which are defined an upward direction 123 and a downward direction 124, such directions merely being so designated based on the orientation of the axis in the figure as illustrated. In the illustrated example, upward direction 123 may correlate to a build direction of a printing apparatus which may be used to additively manufacture an output of the computer-based design process described herein. Vertical reference 122 thus extends along an axis normal to a base plane for additive manufacturing, such as a work surface of a substrate upon which material is deposited or either a bottom or top surface of a powder bed supported by a substrate. Generally, for additive manufacturing processes, downward direction 124 is toward the layers that are deposited earlier, and upward direction 123 is toward the layers that are deposited later. Where subtractive manufacturing is used, reference directions may be defined additionally or alternatively relative to a machining coordinate system. Regardless, vertical reference 122 may be oriented relative to body portion 110 such that the characteristics of the intended manufacturing process or processes compliment, or at least cooperate with, the intended characteristics of the various surfaces of the implant being designed.

An interface side of body portion 110 may be identified before or after holes 118 are created. The interface side in the illustrated example is an upper side of body portion 110, referring to all surfaces of the body portion that would be visible in a plan view of the body portion from a perspective looking in downward direction 124. However, the interface side may be identified as surfaces facing in any direction, or any plurality of directions, relative to vertical reference 122 in other arrangements.

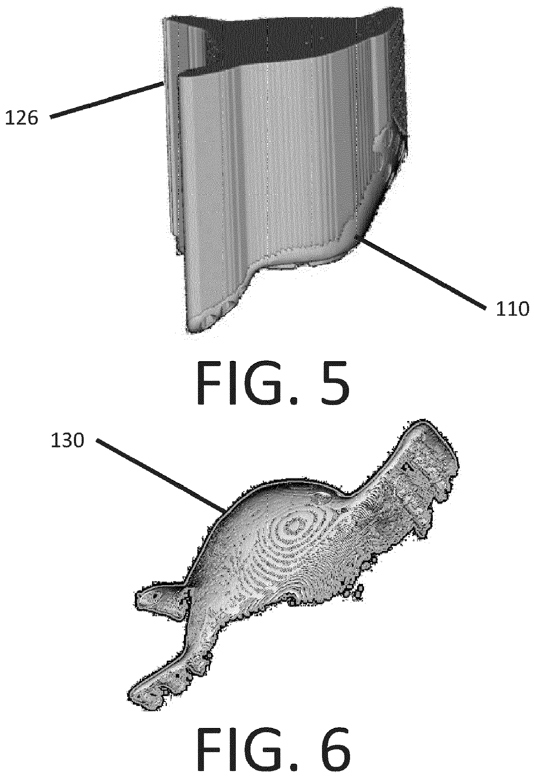

An interface side projection 126 is shown in FIG. 5. Interface side projection 126 is included for illustrative purposes here, but in some software implementations projection functions may be a useful way of identifying the extent of the interface side of the body portion. Interface side projection 126 extends vertically in upward direction 123 from all surfaces of body portion 110 that form part of the interface side of the body portion. In the illustrated example, interface side projection 126 extends only from surfaces of body portion 110 that would be visible in the plan view of the body portion from the perspective looking in downward direction 124, and therefore does not extend under any overhanging features of the body portion. As such, the interface side of the illustrated example includes every point where interface side projection 126 contacts, or would contact if the interface side projection is not actually generated, body portion 110. Stated another way, in the illustrated example, the interface side extends to include every point on body portion 110 that would be visible in a plan view with a perspective looking in the downward direction 124, or every point on the body portion from which an infinitely long line could be drawn in the upward direction 123 without intersecting any other part of the body portion. However, in other examples interface features may be desired on surfaces of body portion 110 located under overhanging features of the body portion, and in such other examples, interface side projection 126 may extend under those overhanging features.

Referring now to FIG. 6, interface model 130, which represents an osseointegrative portion of the implant being designed and which is derived from the above-described identification of the interface side of body portion 110., Interface model 130 is subtracted from body portion 110, except where specifically noted otherwise. Thus, once interface model 130 is given a thickness that extends into body portion 110, the same thickness is subtracted from across the interface side surface of the body portion. Interface model 130 may optionally be given an initial thickness immediately upon creation to aid visualization, and such initial thickness may optionally extend straight downward from the interface side surface of body portion 110 in downward direction 124. Thus, an upper portion of body portion 110 may automatically be subtracted upon creation of interface model 130. However, in the example described below and illustrated, the shape and volume of interface model 130 is manipulated after its creation, and body portion 110 is automatically filled back into any space from which the interface model is removed by a given manipulation. Thus, several steps may exist wherein interface model 130 and body portion 110 are inversely reshaped while an overall net shape of the interface model and body portion 110 is kept constant.

At the completion of the design process described herein, optimized model 150 (see FIG. 14) will be prepared that corresponds to the desired output. A portion of the optimized model 150 that is occupied by final interface model 130B, which is derived from interface model 130, corresponds to a portion of the output that will be fabricated with osseointegrative material or structures. For example, the portions of a fabricated component corresponding to interface model 130 may be porous or have a rough surface. Digital pores or digital surface roughness corresponding to such porous or rough surface may be included in interface model 130 itself. Alternatively, the interface model may simply be the net shape of the osseointegrative portion of the component, and the osseointegrative features may be generated as part of the fabrication process based on instructions stored in a component data computer file or simply component file, e.g., STL file (.stl file extension), VRML (.wrl file extension), AMF (.amf file extension), and G-code (e.g., .nc file extension), that correspond to the fabrication of such osseointegrative features as known to those. For example, creation of printing instructions for additively manufacturing the osseointegrative portion may include tessellating a three-dimensional porous pattern throughout interface model 130, or fabrication of the osseointegrative portion may be carried out in a manner that inherently tends to create pores or surface roughness in the resulting component.

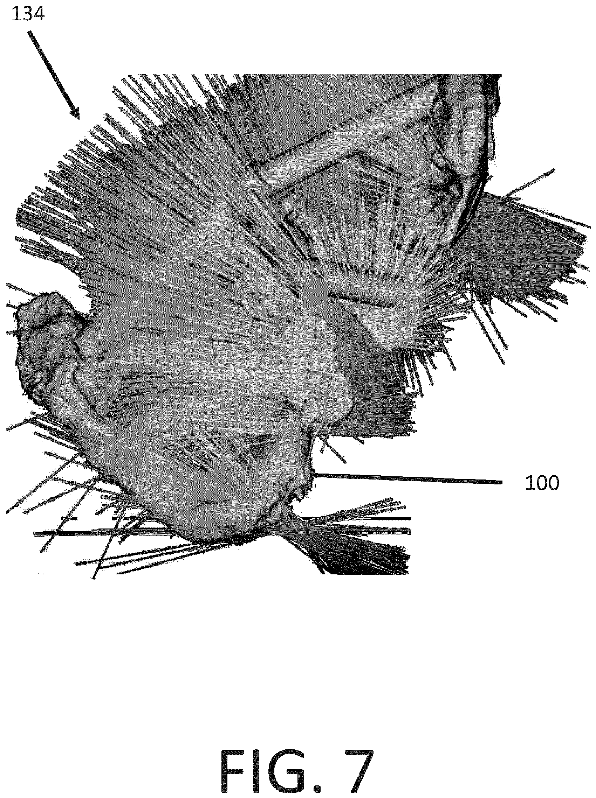

With reference to FIG. 7, the thickness of interface model 130 may be adjusted by reference to bone model 100. A tangent plane between bone model 100 and interface model 130 exists at every point where interface model 130, which is mostly obscured in FIG. 7, contacts bone model 100, or where the interface model nearly contacts the bone model if gaps exist between the interface model and the bone model. Here, "nearly contacts" refers to being within a predefined proximity or being separated by less than a threshold distance. Example threshold distances for near contact include 0 in addition to all quantitative examples provided below as possible values for the thickness of interface model 130. An interface line 134 may be defined normal to each such tangent plane, or at a certain density of such tangent planes per unit area of the interface side of body portion 110. For example, interface lines 134 may be located for each point on a grid extending across the interface side of body portion 110, rather than for every possible point across the interface side. In such examples, the interface side of body portion 110 can be divided into zones, with each zone corresponding to one interface line 134 and containing all points within the interface side nearer to the interface line to which the zone corresponds than any other interface line. Regardless, each interface line 134 extends along an axis of contact between bone model 100 and interface model 130 at a respective location. Depending on the software, interface lines 134 may not need to be visually generated. Thus, reference to interfaces lines 134 herein equally applies to the interface lines themselves, if generated, or the axes of contact or predefined proximity between bone model 100 and interface model 130 at each location upon interface model 130.

A thickness of each location or zone of interface model 130 along a corresponding interface line 134 may be set for that location. At this stage, thickness of the interface model 130 is increased away from bone model 100, generally increasing a distance between points originally identified as part of the interface side of body portion 110 and the transition from the interface model to the body portion. Thus, as thickness of interface model 130 along interface lines 134 increases, thickness of body portion 110 reduces correspondingly.

Thickness added at each location of interface lines 134 may be uniform across interface model 130, to the extent that such thickness would not cause the interface model to extend below the location of any lower surfaces (which may be surfaces on the added thickness side 141 as discussed below) of body portion 110, or may vary across the interface model according to manual inputs or predefined patterns and gradients. The thickness may be added uniformly in all directions in some arrangements, only in directions along interface lines 134 in some other arrangements, only in directions normal to the interface lines in some other arrangements, or uniformly in other predefined directions in still some other arrangements. Where the thickness varies across interface model 130, such variance may be among the foregoing values or generally within the same range as the foregoing values. However, the thickness of interface model, and as a result, the depth of the osseointegrative region of the fabricated implant, may differ from the foregoing examples depending on such factors as physician's preference, feasibility of manufacture, and the portion of bone being replaced by the implant.

If an initial thickness in a direction parallel to vertical reference 122 was given to implant model 130 before the thickness created in directions parallel to interface lines 134, such initial thickness may be discarded when the normal thickness is added. Alternatively, the initial thickness may be retained in any areas not reached by the thickness created in directions parallel to interface lines 134.

Optionally, interface model 130 may be cut to omit any portions of the identified interface side that are unlikely to contact bone in the fabricated implant. Such omitted portions of the interface side may be either any portions that do not directly contact bone model 100 at the intended placement of the implant as represented by the arrangement of initial implant model 108 relative to the bone model as shown in FIG. 1 or any portions that are not within a predetermined distance of the bone model at the intended placement of the implant. Example predetermined distances include distances equal to any of the examples provided above for the thickness of interface model 130 or any of the examples provided below for the thickness of boundary regions 131, 132.

Additionally, a minimum vertical thickness of body portion 110 along the axis of vertical reference 122 may optionally be designated. As interface model 130 is thickened as described above, it may be prevented from extending to any area that would put the vertical thickness of body portion 110 at that location below the designated minimum vertical thickness of the body portion. If a thickness of body portion 110 was below the designated minimum vertical thickness at a given location before the creation of interface model 130, the interface model may be excluded from that location altogether.

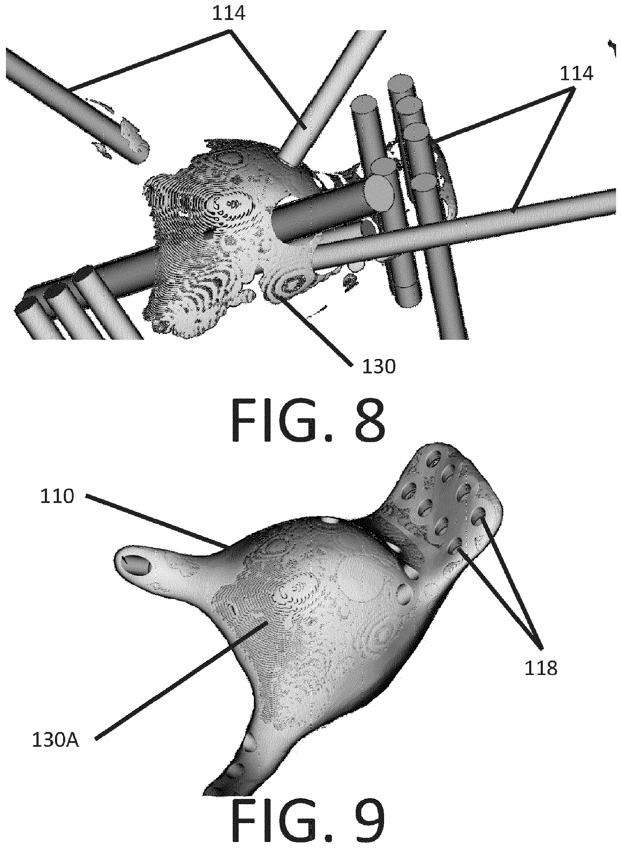

With reference to FIG. 8, interface model 130 may be cut back to provide clearances around separate model components 114, in which the space from which the interface model is removed is replaced by a corresponding part of body portion 110. Such cutting back may be manually input or may be to a uniform, predefined distance from the surface of each separate model component 114. Example suitable distances for cutting back include equal to or about any of equal to or about 1.5 times any of the mean, median, or mode thickness of interface model 130 along interface lines 134, or from 1 to 2 times any of the mean, median, or mode thickness of the interface model along the interface lines. The implant fabricated according to the design that includes body portion 110 and interface model 130 will, as a result of this cutting back of the interface model, have a certain minimum thickness of the base material that the body portion represents around any hole placed according to a separate model component 114. Such minimum thickness may, depending on the material and method of manufacture, improve one or both of the strength and structural integrity of openings through which fasteners are received and improve fabrication speeds and outcomes.

In the illustrated example, such cutting back is performed after holes 118 are placed in body portion 110 and interface model 130 is thickened along interface lines 134, but in other arrangements, the cutting back may occur before either or both of the definition of holes 118 and the thickening along interface lines 134.

As shown in FIG. 9, after thickening of interface model 130 along interface lines 134 and cutting back interface model 130 from separate model components 114 forms modified interface model 130A which is applied to body portion 110. Modified interface model 130A and body portion 110 at this stage have the same net shape together as initial implant model 108. As a result of the above-mentioned exclusions, modified interface model 130A at the stage illustrated in FIG. 9 may include two or more non-continuous portions. Such non-continuous portions would be designated as at least first and second porous sections to be fabricated in the final physical implant, and, upon their identification, would be merged into modified interface model 130A.

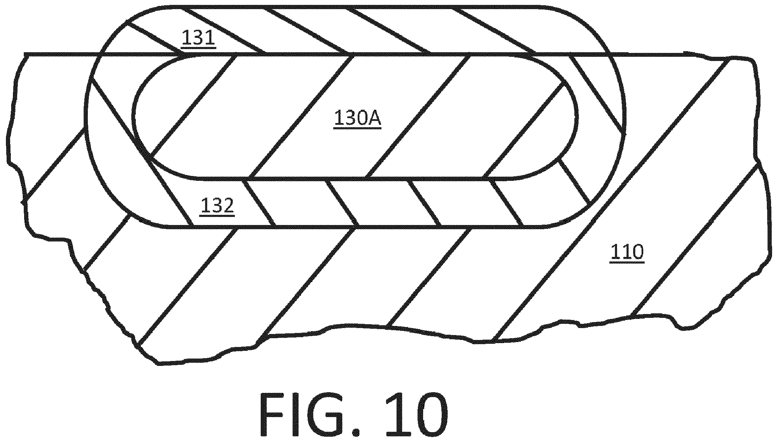

Boundary regions 131, 132 as shown in FIG. 10 may be applied around the space occupied by modified interface model 130A in a state that follows the thickening along interface lines 134 and the cutting back from separate model components 114. Boundary regions 131, 132 of the illustrated example correspond to the volume that would be occupied if a layer of a constant thickness were applied to all surfaces of interface model 130. However, boundary regions 131, 132 may, in other arrangements, have different thicknesses from one another.

Application of boundary regions 131, 132 is optional, and either one or both may be omitted or ignored. However, applied, an outer boundary region 131 is a volume adjacent to interface model 130 that does not intersect body portion 110. Modified interface model 130A may expand to fill outer boundary region 131 and become final interface model 130B such that the osseointegrative portion of the output corresponding to optimized model 150 (see FIG. 14) extends beyond the originally planned net shape for the implant as represented by initial implant model 108. In order for the fabricated implant to fit the patient's bone at the same location that initial implant model 108 fits bone model 100, the output will need to be pushed into place such that the protruding portion of the osseointegrative portion corresponding to outer boundary region 131 will be impressed into the bone. Such compression, especially where the osseointegrative portion is pushed into cancellous bone, will tend to promote osseointegration.

Where inner boundary region 132 is applied, the inner boundary region is a volume adjacent to modified interface model 130A that intersects body portion 110. Inner boundary region 132 may correspond to a transition area in the fabricated implant between the base material and structure of the implant and that of the osseointegrative portion. As such, portions of the output corresponding to inner boundary region 132 may be any type of structure that facilitates secure attachment of the osseointegrative portion. Where the osseointegrative portion is a porous structure, the portions of the output corresponding to inner boundary region 132 may have an intermediate or gradient porosity transitioning from the high porosity of the osseointegrative portion to a relatively lower or lack of porosity of the remainder of the implant. Thus, either one or both of inner boundary region 131 and outer boundary region 132 may be identified as second, third, fourth, or later porous portions and merged into interface model 130.

In the illustrated example, boundary regions 131, 132 are applied to modified interface model 130A as described above. However, in other examples, boundary regions 131, 132 may instead be applied to interface model 130, and the cutting back or exclusions applied to transition interface model 130 to modified interface model 130A may also be applied to boundary regions 131, 132 themselves or whatever features would be populated into boundary regions 131, 132.

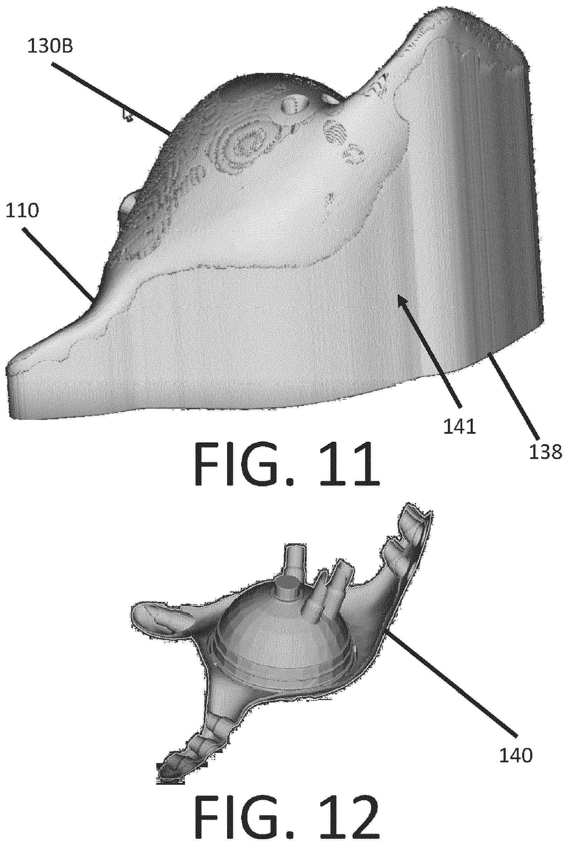

Turning to FIG. 11, added thickness side 141 of body portion 110 may be determined in a similar manner to that which was used to find the interface side as described above with regard to FIG. 5. In the illustrated example, the added thickness side 141 is an underside of body portion 110 and opposite from the interface side. Thus, added thickness side projection 138, which need not be generated in some arrangements, extends up from a lower plane, such as a work surface upon which the output will be built up, to any downward facing surfaces of body portion 110 that are not obscured by other features of the body portion. Added thickness side 141 of body portion 110 will include all surfaces of the body portion contacted by added thickness side projection 138. Thus, added thickness side 141 of body portion 110 includes all surfaces of the body portion that would be visible in a bottom plan view of the body portion from a perspective facing in upward direction 123. However, in other arrangements, added thickness side 141 may have a different directional relationship to the interface side than what is shown and described with regard to the illustrated example, and multiple added thickness sides 141 may be located if appropriate for a planned manufacturing process.

Referring now to FIG. 12, added thickness model 140 may be generated upon added thickness side 141 of body portion 110 after added thickness side 141 is determined. Though FIG. 11 shows interface model 130, the added thickness side 141 may be found and added thickness model 140 may be planned before or after the above-described processes for finding the interface side and creating and planning interface model 130. Added thickness model 140 does not intersect body portion 110. Instead, the thickness of added thickness model 140 extends downward from the added thickness side 141 of body portion 110. Suitable thicknesses for added thickness model 140 will depend on factors including physician and engineer preference, planned fabrication process, and type of implant. However, example thicknesses for added thickness model 140 that would be suitable for the acetabular implant of the illustrated arrangement include values equal to or about 1.0 mm, 1.5 mm, 2.0 mm, 2.5 mm, or 3.0 mm, or either or both of greater than 1.0 mm and less than 3.0 mm. The thickness of added thickness model 140 may be constant across all locations, or may have local variations that may be generated automatically by the computing device, such as according to algorithmic determination of where additional thickness may be structurally beneficial to the fabrication process, or may be created manually.

Added thickness model 140 has the potential to serve multiple purposes. Added thickness model 140 may be fabricated as additional material, optionally of the same, greater, or lesser porosity as the portions of the implant corresponding to body portion 110. Because added thickness model 140 is added to a lower surface of body portion 110, the additional material provided by the added thickness will act as a base upon which the implant itself may be built up. The portion of the output that corresponds to added thickness model 140 may therefore reinforce the implant as it is being constructed. Further, because added thickness model 140 represents excess material, all or some of which must be cut away to put the implant in a finished state, the material of the added thickness can compensate for the poor surface finish and fidelity common to undersides of parts fabricated according to certain additive manufacturing processes which may include or result in build direction issues, pore structure issues, and manufacturing distortion. By machining away the portion of the output that corresponds to added thickness model 140, either manually or by computer numerical control ("CNC"), the lower side of the fabricated implant may have a smoother surface finish than certain additive manufacturing processes are capable of producing on any side of a part. Added material corresponding to added thickness model 140 may therefore be machined away entirely, or may be machined at least out of any holes for separate model components 114 and articulating features such as acetabular socket 114. Here, articulating features refers generally to any features of the implant that will form a joint with another implant or a bone after the implant is placed in the patient's body. Articulating features therefore include sockets, any ball intended for a ball-and-socket joint, and any condyles.

Referring now to FIGS. 13 and 14, optimized model 150 is constructed of final interface model 130B, added thickness model 140, and body portion 110 as modified by the creation and relative placement of interface model 130 and added thickness model 140. The placements of interface model 130 at an upper surface of optimized model 150 and of added thickness model 140 on the lower surface of optimized model 150 as illustrated in FIGS. 13 and 14 work synergistically with a manufacturing process of additive manufacturing followed by machining for an efficient production of an acetabular implant with desired surface properties. According to such a manufacturing process, a raw part according to the optimized model 150 resulting from the above-described design processes is first additively manufactured. During additive manufacturing according to this example, the raw part is constructed in layers normal to the axis of vertical reference 122 such that, for most columns of material extending parallel to the axis of vertical reference 122, material corresponding to added thickness model 140 is constructed first, followed by material corresponding to body portion 110. In columns wherein interface portion 130 is present, osseointegrative structures are then built upon material corresponding to body portion 110. In some examples, osseointegrative structures may be built upon material corresponding to added thickness model 140 in some edge locations though this will not occur in examples where interface model 130 is only permitted to extend to areas where body portion 110 is of at least a minimum vertical thickness. Because the osseointegrative portion covers the top of the additively manufactured raw part, the limitations certain additive manufacturing processes have with regard to producing smooth surface finishes is of little or no consequence. After additive manufacturing is complete, some or all material corresponding to added thickness model 140 is machined away to convert the raw part to a finished implant. Because added thickness model 140 is generally opposite from interface model 130, the material corresponding to the added thickness model will generally cover surfaces of the finished implant corresponding to outer surfaces of the bone that the implant is intended to replace in the illustrated example. The surfaces of the finished implant corresponding to outer surfaces of the bone that the implant is intended to replace may therefore be machined to a smooth finish as the material corresponding to added thickness model 140 is removed, which can be advantageous to the interaction of surrounding portions of the patient's anatomy with the implant as if it were the replaced bone. Such smooth surface finish is particularly useful with respect to recreated articular features, such as the downward facing acetabular socket 144 of the illustrated example.

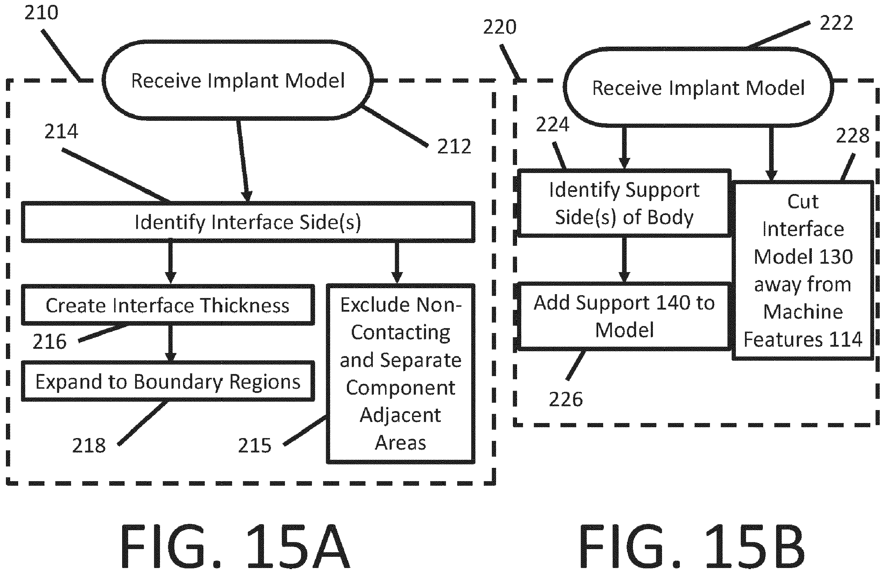

FIG. 15A illustrates an osseointegration optimization process 210, which generally corresponds to the processes described above with regard to FIGS. 5, 7, and 10. At an osseointegration optimization beginning step 212, an initial digital model such as initial implant model 108 of the implant being designed is received and then designated as or converted to body portion 110. At an interface side identification step 214, which occurs after osseointegration optimization beginning step 212, any surfaces of body portion 110 facing in a direction of contact with the bone, which may be selected manually or identified, in some arrangements automatically and in some other arrangements via user input, by computer analysis of a placement of the body portion relative to bone model 100, are designated as part of an interface surface of the body portion, generally as described above with regard to FIGS. 5 and 6. Such identification of the interface surface is a first stage of interface model 130. At an interface thickening step 216, which occurs after interface side identification step 214, a three-dimensional portion of received body portion 110 near the interface side is designated as an interface model 130 generally as described above with regard to FIGS. 6 and 7. At an optional expansion step 218, which occurs after interface thickening step 216, boundary regions 131, 132 are identified as described above with regard to FIG. 10. Interface model 130 may be expanded to fill outer boundary region 131, which does not intersect body portion, and inner boundary region 132, which intersects the body portion, may be designated for fabrication as a connecting or transitional structure between the main body of the implant and the interface portion of the implant. At any time after interface side identification step 214 and before finalization of the optimized model 150, area excluding step 215 may optionally be executed. At area excluding step 215, the surfaces designated as the interface side or porous volumes derived from such surfaces are updated to exclude any surfaces or areas that do not contact bone model 100, or are spaced from a nearest surface of the bone model by more than a threshold distance, when the implant model is placed relative to the bone model in a location corresponding to the intended placement of the fabricated implant relative to the patient's bone. Excluding step 215 may also include excluding the surfaces designated as the interface side or porous volumes derived from such surfaces from areas less than a threshold distance away from holes provided for separate model components 114 or holes provided for the separate model components. Any downstream interface features, such as a volume of interface model 130 or boundary regions 131, 132, that may have been extrapolated from the surface areas excluded during excluding step 225 are reverted to being either empty space or part of body portion 110 at the end of the excluding step.

FIG. 15B illustrates a structural optimization process 220, which generally corresponds to the processes described above with regard to FIGS. 8, 11, and 12. At structural optimization beginning step 212, a digital model of the implant being designed, such as body portion 110 in combination with interface portion 130, is received. Added thickness side identification step 224 and added thickness modeling step 226 may be executed before, after, or during creation of interface model 130. Thus, structural optimization beginning step 212 may cease after only implant body 110 is received, or may continue until or resume upon receipt of interface model 130, which may occur after added thickness modeling step 226.

At added thickness side identification step 224, which may occur after at least body portion 110 is received, surfaces of body portion 110 facing toward a direction from which support from sacrificial material may be advantageous during fabrication are designated as added thickness side 141 as described above with regard to FIG. 11. If interface model 130 exists, surfaces of interface model 130 facing in the direction from which support from sacrificial material may be advantageous optionally may be designated as part of the added thickness side 141. At added thickness modeling step 226, added thickness model 140 corresponding to structure to be fabricated as sacrificial material is added upon the added thickness side 141 identified in added thickness side identification step 224. Added thickness modeling step 226 may be generally alike to processes described above with regard to FIGS. 11 and 12.

A separate model component clearance step 228 may be executed before, after, between, or during any of added thickness side identification step 224, added thickness modeling step 226, interface thickening step 216, and expansion step 218, but must be executed after added thickness side identification step 214. At separate model component clearance step 228, interface model 130, at whatever stage of development interface model 130 may be, is excluded from separate model components 114 by a predetermined distance as described above with regard to FIG. 8. Areas previously occupied by body portion 110 and from which interface model 130 is excluded during separate model component clearance step 228 are reverted to being part of the body portion during the separate model component clearance step 228.

Osseointegration optimization process 210 and structural optimization process 220 according to the illustrated example are somewhat mutually interdependent due to the effects each has on the outcome of the other. Local reinforcements may be added to body portion 110 to support osseointegrative features, and osseointegrative features of the model are excluded from areas where stronger structures are needed or where the osseointegrative features would present undue manufacturing difficulty. A design method having such interdependence between processes serving different considerations has the potential to result in implants that are satisfactory from both medical and engineering perspectives.

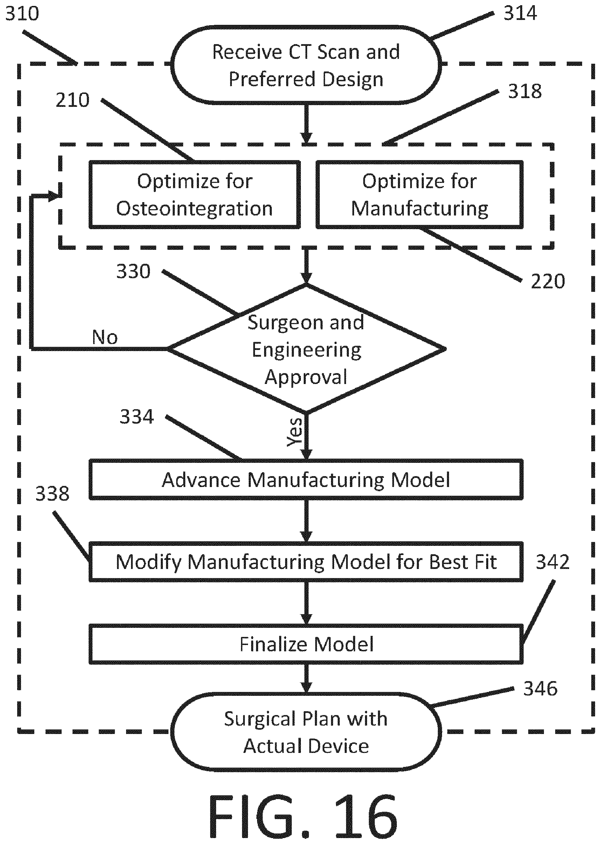

Osseointegration optimization process 210 and structural optimization process 220 may be integrated into a "digital thread" process 310 illustrated in FIG. 16 according to which medical professionals and engineers may cooperate to optimize orthopedic implants. Digital thread process 310 may be used to create manufacturing plans for patient-specific implants or implants intended to become off-the-shelf products.

At an input step 314, the digital thread begins with three-dimensional models of an implant and a bone, such as initial implant model 108 and bone model 100 as illustrated in FIG. 1. Initial implant model 108 may be placed and shaped according to any computer-based implant designing or surgical planning processes that produce, at some stage, a computer model of an implant placed relative to a computer model of a bone at a location corresponding to the intended final placement of an actual implant within a patient. Further, though FIG. 16 refers to a CT scan by way of example, bone models 100 acquired by any technology may be received as an input into digital thread process 310.

Following input step 314, initial implant model 108 becomes body portion 110 and proceeds through an optimization stage 318. Optimization stage 318 includes osseointegration optimization process 210 and structural optimization process 220 and generally corresponds to the procedures described above with regard to FIGS. 1-15B.

Optimized computer model 150 produced by optimization stage 318, which may be a body portion 110, final interface model 130B, and added thickness model 140 arranged as shown in FIG. 14, is presented for consideration at a review step 330. At review step 330, medical and manufacturing professionals consider the optimized model for factors such as likelihood of successful integration and feasibility of manufacture. Review step 330 thus presents an opportunity for interdisciplinary collaboration to supplement any rote or automated aspects of optimization stage 318. If the reviewing professionals determine that the model would be ready for fabrication with no or only minor manual adjustments, the model is advanced for manufacturing at advancement step 334. If the reviewing professionals instead determine that the optimized model is unsatisfactory, some or all of optimization stage 318 is repeated. Numerical constants, such as thicknesses for any of interface model 130, clearance around separate model components 114, boundary regions 131, 132, or added thickness model 140, may be changed upon revisiting optimization stage 318. Additionally, local variations in thickness or geometry may be added manually at any time within repeat passes through optimization stage 318, such as adjusting thicknesses of interface model 130 or added thickness model 140 up or down overall or locally, or extending or excluding the interface model or added thickness model from the locations to which those features extend at the end of the optimization stage.

After the model is advanced for manufacturing at advancement step 334, the model may optionally pass through a modification step 338. During modification step 338, manual or automated adjustments to any geometry of the model are made for medical or manufacturing purposes as necessary. Adjustments during modification step 338 are typically, though not necessarily, relatively minor in nature. At the conclusion of modification step 338, or at the conclusion of advancement step 334 if modification step 338 is skipped, the model is finalized for manufacturing in finalizing step 342. Either one or both of the finalized digital model itself and an actual implant fabricated according to optimized model 150, which includes body 110, final interface model 130B, and added thickness model 140, may be used to create a new or updated surgical plan in planning step 346.

Although the concepts herein have been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A method of preparing a computer-generated component file for fabricating an orthopedic implant comprising steps of:
setting, via a computer processor, first and second select sections of an initial implant model (108) of a computer-aided design model to first and second model porous sections (130) and leaving a remaining section (110) of the initial implant model, wherein all regions defining the first and the second select sections are spaced not more than a uniform preset distance from a patient-specific bone model (100) of the computer-aided design model;
merging, via the computer processor, the first and the second model porous sections with the remaining section of the initial implant model to form at least a portion of a final implant model (150) of the computer-aided design model; and
storing, via the computer processor, the final implant model in a component file configured to be accessed by a computer-aided manufacturing machine for use in fabricating the orthopedic implant, at least a portion of the orthopedic implant corresponding to the final implant model.

2. The method of claim 1, further comprising a step of removing, before the setting step and via the computer processor, the first and second select sections from the initial implant model, wherein the remaining section of the initial implant model remains following the removing step.

3. The method of claim 1 or claim 2, wherein the first and the second model porous sections have a first model porous thickness, further comprising a step of:
setting, via the computer processor, a third select section of the initial implant model to a third model porous section, wherein all regions defining the third select section are spaced not more than the preset distance from the patient-specific bone model as measured uniformly, wherein first and second model implant thicknesses of the initial implant model taken along respective first and second lines through the first and the second select sections include at least a preset minimum model continuous solid thickness and a third model implant thickness of the initial implant model taken along a third line through the third select section includes less than the preset minimum model continuous solid thickness, the third model porous section having a second model porous thickness equal to a difference between the third model implant thickness and the preset minimum model continuous solid thickness when the third model implant thickness is greater than the preset minimum model continuous solid thickness or is otherwise zero,
wherein the merging step comprises merging, via the computer processor, the third model porous section with the remaining section of the initial implant model to form at least the portion of the final implant model.

4. The method of claim 3, further comprising a step of removing, before the third select section setting step and via the computer processor, the third select section from the initial implant model.

5. The method of claim 2 or claim 4, further comprising a step of receiving, via the computer processor, an input instruction, wherein each of the removing, setting, and merging steps occur automatically in response to the input instruction.

6. The method of any one of claims 1-5, wherein the first and the second select sections include first and second digital surfaces contacting the patient-specific bone model.

7. The method of any one of claim 1, claim 2, and claim 6 when depending from claim 1 or claim 2, further comprising a step of removing from the select sections, before the setting step and via the computer processor, any regions of the initial implant model that are not within a porous thickness distance of any point on any surface of the initial implant model that is within an interface proximity of a bone model.

8. The method of any one of claims 1-7, further comprising a step of extending, via the computer processor, the model porous sections beyond the initial implant model.

9. The method of any one of claims 1-8, further comprising a step of setting, via the computer processor, a boundary region of the initial implant model, the boundary region being contiguous with one or more of the model porous sections as a region of gradient porosity.

10. The method of any one of claims 1-9, further comprising a step of extending, via the computer processor, a model added thickness layer having a predetermined model thickness to a side of the initial implant model facing away from the bone model.

11. The method of claim 10, further comprising steps of:
fabricating a component according to the final implant model; and
removing at least some material from the component corresponding to the model added thickness layer to define at least a portion of the orthopedic implant.

12. The method of any one of claims 1-11, wherein the first and the second select section setting step includes sending instructions, via the computer processor, to a computer-aided design model to associate a porosity with the first and the second model porous sections of the initial implant model.

13. A method of replacing a portion of a bone comprising steps of:
removing one or more bone portions from one or more bones to leave, respectively, a remaining first bone portion or a remaining first plurality of bone portions; and
placing an implant having a plurality of irregularly shaped porous portions and a plurality of irregularly shaped solid portions each separating at least portions of respective pairs of the plurality of irregularly shaped porous portions against only the respective remaining first bone portion or first plurality of bone portions such that none of the plurality of irregularly shaped porous portions includes a region located more than a preset distance from at least one bone portion of the respective remaining first bone portion or first plurality of bone portions, the irregularly shaped porous portions and the irregularly shaped solid portions defining all bone-facing surface portions of the implant.

14. An orthopedic implant comprising:
a first implant structure corresponding to a first implant model (150) of a computer-aided design model, the computer-aided design model being prepared by steps of:
forming, via a computer processor, a patient-specific bone model (100) of a first bone portion or a first plurality of bone portions within the computer-aided design model; and
forming, via the computer processor, the first implant model (150) with porous regions (130B) and solid regions (110), the porous regions being formed only at locations of the first implant model within a preset distance from the bone model.

15. The orthopedic implant of claim 14 wherein the porous regions of the first implant model are formed at all locations of the first implant model within the preset distance from the bone model with the exceptions of i) regions surrounding digital holes (118) corresponding to holes configured for receiving shafts of separate objects separate from the first implant structure and ii) regions within a minimum continuous solid thickness zone of the first implant model, the minimum continuous solid thickness zone having a boundary (131) on a digital opposing surface of the first implant model opposite a digital bone-facing surface of the first implant model facing the bone model and having a thickness corresponding to a minimum continuous solid thickness preset within the first implant model.
